# EUROPEAN PATENT APPLICATION

(11) **EP 2 407 156 A2**
(43) Date of publication of application: **18.01.2012**
(21) Application number: 11174030.4
(22) Date of filing: 14.07.2011
(51) Int. Cl.: A61K 9/10, A61K 47/10, A61K 47/26, A61K 47/44, A61K 9/16

(54) **Aqueous coacervate compositions suitable for making powders and water-soluble formulations of biologically-active agents**

(30) Priority: 14.07.2010 GB 1011821
(71) Applicant: Universiteit Gent, 9000 Gent (BE)
(72) Inventor: Remon, Jean Paul, 9090 Melle (BE); Vervaet, Chris, 8870 Izegem (BE); Da Fonseca Antunes, Andre Bruno, 3030-461 Coimbra (PT)
(74) Representative: Bird Goën & Co

(57) **Abstract**

This invention relates to an aqueous gel-free two-phase coacervate composition comprising a coacervate phase and an equilibrium water phase, comprising a mixture of:
(a) a poorly water-soluble bio-active agent,
(b) a tensio-active system with a HLB from 8 to 18 and consisting of non-ionic tensio-active agents,
(c) one or more water-soluble carriers for said poorly water-soluble bio-active agent, said carriers being selected from the group consisting of polyols, and
(d) water.

Such coacervate compositions may be dried into solid dosage forms from which rapid release of the bio-active agent can be obtained.

## Description

### FIELD OF THE INVENTION

The present invention relates to the formulation of poorly water-soluble biologically-active agents such as therapeutic drugs, pesticides, fertilizers, cosmetics and the like. The present invention especially relates to the formulation of human and veterinary therapeutic drugs belonging to Class II or Class IV of the Bio Classification System (BCS) showing a low oral bioavailability due to their low water solubility. More specifically the present invention relates to aqueous coacervate systems including poorly water-soluble biologically-active agents such as therapeutic drugs, pesticides, fertilizers, cosmetics and the like. The present invention also relates to biologically-active agent powders and granules that can be produced, e.g. by drying, from such aqueous coacervate systems and that are easily water-soluble or water-dispersible and that are able to achieve, upon administration to a human or animal (therapeutic drugs and cosmetics) or upon dispersion to a locus to be treated (pesticides and fertilizers), a desired release kinetics e.g. an immediate release.

### BACKGROUND OF THE INVENTION

The Biopharmaceutical Classification System (hereinafter referred as BCS) according to G. Amidon et al. in Pharm. Res. (1995) 12:413-420 provides for two classes of poorly soluble drugs, i.e. Class II and Class IV, and a class of highly soluble drugs, i.e. Class I. The BCS pertains both to the human therapeutic field and to the veterinary art. According to M. Martinez et al., Applying the Biopharmaceutical Classification System to Veterinary Pharmaceutical Products (Part I: Biopharmaceutics and Formulation Consideration) in Advanced Drug Delivery Reviews (2002) 54:805-824, a drug substance should be classified as highly soluble when the highest dose strength is soluble in at most 250 ml of aqueous media over the pH range 1-7.5.

Human therapeutic drugs or veterinary drugs belonging to class II or IV of the BCS are known to the skilled person not to be very efficacious after oral administration whatever the oral dosage form (tablets, pellets, liquids, ...) because of a decreased bioavailability at the place of absorption, itself often due to the low aqueous solubility of the drug.

Veterinary drugs for pigs, poultry and other cattle or livestock animals are often administered via feed or drinking water because of the fact that a large number of animals can be medicated at the same time (mass medication). Especially drinking water and to a lesser extent feed medication are very popular. Drinking water medication offers several advantages and a few disadvantages in relation to prophylactic and therapeutic treatment. Important advantages are the low cost of organization and the easiness of administration. Immediate therapeutically care of all sick or endangered animals in the flock and - if necessary - a quick change to another drug and/or dose are possible. An important advantage is that all sub-clinical sick animals and animals in the incubation period are also treated, avoiding further spreading of the disease in the flock. The main disadvantages of animal mass medication via drinking water are (1) possible variations in drug uptake between the animals and (2) the fact that most drugs are not water-soluble or water-dispersible enough or their water-solutions or water-dispersions are not stable enough along time. Because of the practical and organizational importance of drinking water based therapy for animals, water-soluble and water-dispersible drug formulations have an important contribution in the veterinary industry.

The same concern about the poor bioavailability of certain drugs is also more and more applicable to human therapy. This is especially due to the fact that most newly developed human drugs tend to be poorly water-soluble, i.e. belong to Class II or Class IV of the BCS. The same concern about poor bioavailability is also applicable to new types of pesticides and fertilizers which tend to be poorly water-soluble and which, if properly dispersed at the locus to be treated in a more water-soluble or water-dispersible form, would highly contribute to improved treatment efficiency and/or decreased damages to the environment.

Coacervation can be considered as a phenomenon in which aqueous colloidal solutions separate - upon alteration of the thermodynamic condition of state - into two liquid phases, one being rich in colloid (i.e. the coacervate phase) and the other one containing little colloid (i.e. the equilibrium water phase). Coacervation is readily distinguishable from precipitation, which occurs in colloidally unstable systems and where a coagulum of flocs is formed.

A few gelatin-based or hydrogel-based aqueous coacervate systems are known in the art, however they are not practically suitable for most administration modes of human or veterinary poorly water-soluble therapeutic drugs, due to the physical characteristics associated with gelified forms. Therefore there is still a need in the art for gel-free coacervate compositions including one or more poorly water-soluble biologically active agents.

### SUMMARY OF THE INVENTION

Unexpectedly, the combination in suitable proportions of a poorly water-soluble biologically-active agent, a non-ionic tensio-active agent system, one or more water-soluble biocompatible carriers (such as maltodextrins, polyols and fructose, or mixtures thereof) and water is able to result in the formation of aqueous gel-free two-phase coacervate compositions wherein a major part of the poorly water-soluble biologically-active agent is present in the coacervate phase. Such compositions are suitable for the administration of a therapeutic drug or cosmetic agent to humans and animals, and for the dispersion of a pesticide or fertilizer at a locus to be treated. The coacervate compositions of this invention, or the coacervate phase thereof, can also be used for the production of solid dosage forms via spray-drying, freeze-drying or any other drying technique such as, but not limited to, granulation which does not harm the biologically-active agent, to obtain a water soluble or water-dispersible powder, which can be easily applied or administered to a locus to be treated with said biologically-active agent, e.g. a drinking water medication for humans or animals. If desired, this water soluble or water-dispersible powder can also be further processed into other solid dosage forms such as tablets, capsules, sachets and the like. This water soluble or water-dispersible powder also shows ability to provide an immediate release dissolution profile after administration together with water. Furthermore the aqueous biphasic coacervate systems of this invention, as well as solid dosage forms derived therefrom after drying, are stable within a broad temperature range from about 5°C to about 45°C and under broad relative humidity conditions, therefore can be stored under such conditions without a loss of their rapid bioactive agent release characteristics.

### BRIEF DESCRIPTION OF THE DRAWINGS

Figure 1 shows results of a dissolution test according to U.S. Pharmacopeia standard 27 performed on a febantel powder formulation according to the invention in comparison with febantel reference formulations.
Figure 2 shows results of *in vivo* administration of a febantel powder formulation according to the invention to pigs.

### DETAILED DESCRIPTION OF THE INVENTION

According to a first embodiment, the present invention relates to a coacervate system for the formulation of relatively low loadings (concentrations) of poorly water-soluble biologically-active agents. More specifically this first embodiment of the present invention relates to an aqueous gel-free two-phase coacervate composition comprising a coacervate phase and an equilibrium water phase, wherein said composition comprises, or alternatively consists essentially of, per 100 parts by weight, a mixture of:
(a) from 2 to 10 parts by weight of a poorly water-soluble biologically-active agent,
(b) from 10 to 30 parts by weight of a polysaccharide-free tensio-active system with a HLB from 8 to 18 and consisting essentially of one or more non-ionic tensio-active agents,
(c) from 10 to 25 parts by weight of one or more water-soluble biocompatible carriers selected from the group consisting of maltodextrins with a molecular weight below 1,800, erythritol, xylitol, sorbitol, mannitol, maltitol, isomalt, lactitol, fructose, oligofructose, polyfructose, and mixtures thereof, and
(d) from 40 to 70 parts by weight of water,
wherein the weight ratio (c)/(b) of the water-soluble carriers to the non-ionic tensio-active agents is from 0.3/1 to 2.5/1.

According to a second embodiment, the present invention relates to a coacervate system for the formulation of relatively high loadings (concentrations) of poorly water-soluble biologically-active agents. More specifically this second embodiment of the present invention relates to an aqueous gel-free two-phase coacervate composition comprising a coacervate phase and an equilibrium water phase, wherein said composition comprises, or alternatively consists essentially of, per 100 parts by weight, a mixture of:
(e) more than 10 parts and up to 30 parts by weight of a poorly water-soluble biologically-active agent,
(f) more than 30 parts and up to 70 parts by weight of a polysaccharide-free tensio-active system with a HLB from 8 to 18 and consisting essentially of one or more non-ionic tensio-active agents,
(g) from 2 to less than 10 parts by weight of one or more water-soluble biocompatible carriers selected from the group consisting of maltodextrins with a molecular weight below 1,800, erythritol, xylitol, sorbitol, mannitol, maltitol, isomalt, lactitol, fructose, oligofructose, polyfructose, and mixtures thereof, and
(h) from 20 to less than 40 parts by weight of water,
wherein the weight ratio (g)/(f) of the water-soluble carriers to the non-ionic tensio-active agents is from 0.005/1 to less than 0.3/1.

The term gel-free, as used with respect to the coacervate compositions of the first and second embodiments of the present invention, refers to the absence of gel-forming components such as, but not limited to, gelatin, hydrogel, egg yolk and egg white.

Within the framework of the above definitions of the first and second embodiments of the present invention, and merely using the exemplary teachings detailed below, the skilled person can determine via routine experimental optimisation work, which specific weight proportions of the four required components are most suitable for achieving a gel-free coacervate system, depending upon the amount and the type (e.g. chemical and/or physical characteristics such as water-solubility, kind and number of chemical functionalities, etc.) of the poorly soluble biologically-active agent to be formulated.

Depending upon the intended route of administration or the intended mode of application of the biologically-active agent formulation, the proportion by weight of the poorly water-soluble biologically-active agent in the mixture of the first embodiment of the present invention may be at most 5 parts by weight per 100 parts by weight of the coacervate composition. Similarly the proportion by weight of the poorly water-soluble biologically-active agent in the mixture of the second embodiment of the present invention may be at least 15 parts by weight and/or at most 25 parts by weight per 100 parts by weight of the coacervate composition.

In a specific embodiment of the present invention the tensio-active system (b) or (f) is preferably free from high molecular weight polysaccharides such as dextran (molecular weight above 10,000). In another specific embodiment of the present invention the tensio-active system (b) or (f) exhibits a hydrophilic-lipophilic balance (HLB) from 10 to 16.

The poorly water-soluble biologically-active agent (a) or (e) present in the gel-free two-phase coacervate compositions according to the above described embodiments of the present invention may be a therapeutic drug for human or veterinary use, a cosmetic agent, a pesticide, a fertiliser, a herbicide, a plant growth regulator, a crop treatment agent, an anti-microbial agent (in particular a fungicide or a bactericide), admissible for use in plants, animals and/or humans. Thus the biologically active compositions according to this invention may be for pharmaceutical use, cosmetic use, veterinary use or for plant treatment.

When the poorly water-soluble biologically-active agent (a) or (e) is a poorly soluble drug or cosmetic agent, it preferably belongs to Class II or Class IV of the BCS. As known to those skilled in the art, such human or veterinary drugs belong to various therapeutic classes including, but not limited to, β-blockers, calcium antagonists, ACE inhibitors, sympatho-mimetic agents, hypoglycaemic agents, contraceptives, α-blockers, diuretics, anti-hypertensive agents, anti-psoriatics, bronchodilators, cortisones, anti-mycotic agents, salicylates, cytostatic agents, antibiotic agents, virustatic agents, antihistamines, UV-absorbers, chemotherapeutics (e.g. antiproliferative agents), antiseptics, estrogens, scar treatment agents, anti-fungal agents, antibacterial agents, anticoccidials, antifolate agents, cardiovascular agents, nutritional agents, antispasmodics, analgesics, antipyretics, anti-inflammatory agents, coronary vasodilators, peripheral vasodilators, anti-tussive agents, muscle relaxants, tranquilisers, antiarrythmic agents, anticoagulants, anti-emetics, anthelmintic agents, expectorants, anti-diabetic agents, immunosuppressants, central nervous system agents (e.g. against Alzheimer or Parkinson diseases), analgesics, hypolipidemic agents and the like.

For instance the present invention is suitable for formulating therapeutically active drugs or cosmetic agents belonging to various chemical or therapeutic classes such as, but not limited to, the following: benzimidazole-based anthelmintics, albendazole, fenbendazole, flubendazole, oxfendazole, thiabendazole, paclitaxel, febantel, pyrantel, florfenicol, acebutolol, acetohexamide, acetylcysteine, acetylsalicylic acid, acyclovir, ajamaline, alendronate, alfuzosine, alprazolam, alfacalcidol, allantoin, allopurinol, alverine, ambroxol, amikacin, amlodipine, amiloride, aminoacetic acid, amiodarone, amitriptyline, amlodipine, amoxicillin, ampicillin, amylobarbitone, ascorbic acid, aspartame, astemizole, atenolol, beclomethasone, benserazide, benzalkonium hydrochloride, benzocaine, benzoic acid, betamethasone, bezafibrate, biotin, biperiden, bisoprolol, bromazepam, bromhexine, bromocriptine, budesonide, bufexamac, buflomedil, buspirone, caffeine, camphor, captopril, carbamazepine, carbidopa, carboplatin, cefachlor, cefalexin, cefatroxil, cefazolin, cefixime, cefotaxime, ceftazidime, ceftriaxone, cefuroxime, cephalosporins, cetirizine, chloramphenicol, chlordiazepoxide, chlorhexidine, chlorpheniramine, chlortalidone, choline, cyclosporin, cilastatin, cimetidine, ciprofloxacin, cisapride, cisplatin, citalopram, clarithromycin, clavulanic acid, clomipramine, clonazepam, clonidine, clotrimazole, clozapine, codeine, cholestyramine, cyproterone, desogestrel, dexamethasone, dexpanthenol, dextromethorphan, dextropropoxiphen, diazepam, diclofenac, digoxin, dihydrocodeine, dihydroergotamine, dihydroergotoxin, diltiazem, diphenhydramine, dipyridamole, dipyrone, disopyramide, domperidone, dopamine, doxycycline, enalapril, ephedrine, epinephrine, ergocalciferol, ergotamine, erythromycin, estradiol, ethinyl estradiol, etoposide, *Eucalyptus globulus,* famotidine, felodipine, fenofibrate, fenoterol, fentanyl, fluconazole, flunarizine, fluorouracil, fluoxetine, flurbiprofen, furosemide, gallopamil, gemfibrozil, *Ginkgo biloba,* glibenclamide, glipizide, griseofulvin, guaifenesin, haloperidol, hydrochlorothiazide, hydrocodone, hydrocortisone, hydromorphone, ibuprofen, imipenem, indomethacin, isosorbide dinitrate and mononitrate, isotretinoin, ketotifen, ketoconazole, ketoprofen, ketorolac, labetalol, lactulose, lecithin, levocarnitine, levodopa, levoglutamide, levonorgestrel, levothyroxine, lidocaine, lipase, imipramine, lisinopril, loperamide, lorazepam, lovastatin, medroxyprogesterone, menthol, methotrexate, methylprednisolone, metoclopramide, metoprolol, miconazole, midazolam, minocycline, minoxidil, misoprostol, morphine, N-methylephedrine, naftidrofuryl, naproxen, neomycin, nicardipine, nicergoline, nicotinamide, nicotine, nicotinic acid, nifedipine, nimodipine, nitrazepam, nitrendipine, nizatidine, norethisterone, norfloxacin, norgestrel, nortriptyline, nystatin, ofloxacin, omeprazole, ondansetron, pancreatin, panthenol, pantothenic acid, paracetamol, paroxetine, phenobarbital, pentoxifylline, phenoxymethylpenicillin, phenylephrine, phenylpropanolamine, phenytoin, physostigmine, piroxicam, polymyxin B, povidone iodine, pravastatin, prazepam, praziquantel, prazosin, prednisolone, prednisone, propafenone, propranolol, proxyphylline, pseudoephedrine, pyridoxine, quinidine, ramipril, ranitidine, reserpine, retinol, riboflavin, rifampicin, risperidone, rutoside, saccharin, salbutamol, salcatonin, salicylic acid, simvastatin, somatotropin, sotalol, spironolactone, sucralfate, sulbactam, sulfamethoxazole, sulfasalazine, sulpiride, tamoxifen, tegafur, teprenone, terazosin, terbutaline, terfenadine, tetracaine, tetracycline, theophylline, thiamine, ticlopidine, timolol, tranexamic acid, tretinoin, triamcinolone acetonide, triamterene, triazolam, trimethoprim, troxerutin, valproic acid, verapamil, folinic acid, zidovudine and zopiclone. The present invention is also suitable for formulating poorly water-soluble organic and inorganic addition salts (including acid addition salts and base addition salts) and poorly water-soluble solvates (such as, but not limited to, hydrates and alcoholates) of the above-mentioned therapeutic agents. For instance, acid-addition salts include hydrochlorides, hydrobromides, sulfates, phosphates, carbonates, monocarboxylic and dicarboxylic acid salts and sulfonates; base addition salts include alkali and alkaline-earth metal salts, ammonium and alkylammonium salts, arginine, lysine, choline or procaine salts, etc.

In a specific embodiment of the present invention, the poorly water-soluble biologically active agent is a therapeutic drug with a molecular weight from 200 to 800 or, preferably, from 200 to 410.

In a specific embodiment of the present invention, the poorly water-soluble biologically active agent is a nitrogen-containing drug such as, but not limited to, febantel. In another specific embodiment of the present invention, the poorly water-soluble biologically active agent is a nitrogen-free drug such as, but not limited to, fenofibrate and ketoprofen.

In a specific embodiment of the present invention, the poorly water-soluble biologically active agent is a drug capable of lowering plasma cholesterol levels. In a more specific embodiment of the present invention, the poorly water-soluble biologically active agent is a fibrate which may be a nitrogen-free fibrate such as, but not limited to, ciprofibrate, fenofibrate, gemfibrozil and analogs thereof, or may be a nitrogen-containing fibrate such as bezafibrate. In another specific embodiment of the present invention, the poorly water-soluble biologically active agent is a statin which may be a nitrogen-free statin such as, but not limited to, lovastatin, mevastatin, pravastatin and analogs thereof, or may be a nitrogen-containing statin such as rosuvastatin, atorvastatin, cerivastatin, fluvastatin, pitavastatin and analogs thereof.

The present invention is especially suitable for the formulation of therapeutically active ingredients (drugs) having a water-solubility below about 2.5 mg/ml, even between 0.1 and 1 mg/ml (i.e. " very slightly soluble " as defined in the United States Pharmacopeia), even below 0.1 mg/ml (i.e. " practically insoluble " as defined in the United States Pharmacopeia) or below 0.02 mg/ml, even below about 5 µg/ml and may even have a water-solubility as low as about 0.2 µg/ml, at room temperature and physiological pH. Non-limiting examples of such drugs include for instance febantel, hydrochlorothiazide, nimodipine, flufenamic acid, mefenamic acid, bendroflumethiazide, benzthiazide, ethacrinic acid, nitrendipine and diaminopyrimidines, including enantiomers thereof, organic and inorganic addition salts (including acid salts and base salts) thereof, and solvates (such as hydrates and alcoholates) thereof. Suitable examples of such poorly soluble diaminopyrimidines include, without limitation, 2,4-diamino-5-(3,4,5-trimethoxy-benzyl) pyrimidine (trimethoprim), 2,4-diamino-5-(3,4-dimethoxybenzyl) pyrimidine (diaveridine), 2,4-diamino-5-(3,4,6-trimethoxybenzyl) pyrimidine, 2,4-diamino-5-(2-methyl-4,5-dimethoxybenzyl) pyrimidine (ormetoprim), 2,4-diamino-5-(3,4-dimethoxy-5-bromobenzyl) pyrimidine, 2,4-diamino-5-(4-chloro-phenyl)-6-ethylpyrimidine(pyrimethamine), and analogues thereof.

As demonstrated in one example below, the major part (that is more than 50%) of the poorly water-soluble biologically-active agent (a) or (e) is usually advantageously included in the coacervate phase of the aqueous gel-free two-phase coacervate composition of the present invention. Depending upon parameters such as the particular poorly water-soluble biologically-active agent, the type and proportions of the other required components, the proportion of the poorly water-soluble biologically-active agent which is included in the coacervate phase may be at least 70%, or at least 80%, or at least 90%, and may reach up to 100%.

The weight ratio of the water-soluble biocompatible carrier to the non-ionic tensio-active agent in the mixture may influence the easiness of formation or the behavior of the aqueous gel-free two-phase coacervate composition according to the present invention and may be selected at will within the ranges stated in the above general expression of each embodiment.

The non-ionic tensio-active agent present in the component (b) or (f) of the aqueous gel-free two-phase coacervate compositions of both embodiments of the present invention may be selected from a broad class of chemical compounds well known to the skilled person, e.g. may be selected from the group consisting of cholesterol esters, ethylene glycol fatty acid esters, ethoxylated esters, (poly)glycerol fatty acid esters (e.g. stearate), mono- and diglycerides of fatty acids, poloxamers (e.g. polyoxyethylkene/polyoxypropylene copolymers with a molecular weight from 1,000 to 7,000 such as the Pluronic commercial grades), polyoxyethylene alkyl ethers (e.g. cetyl or cetostearyl), polyoxyethylene castor oil derivatives, polyoxyethylene sorbitan mono-fatty acid esters (also known as polysorbates, e.g. the commercial grade Tween 80 illustrated in some examples below, but also Tween 20, Tween 40, Tween 60 and Tween 85), propyleneglycol esters, sorbitan fatty acid esters (e.g. derived from lauric, palmitic or stearic acid, such as the commercial grades Span 20, Span 40, Span 60, Span 65, Span 80 and Span 85), and mixtures thereof. When more than one non-ionic tensio-active agent is used, their respective proportions should be such that the hydrophilic-lipophilic balance (HLB) of the tension-active system is from 8 to 18. Any bio-compatible or pharmaceutically acceptable grade of an above-mentioned non-ionic tensio-active agent may be used as a component (b) or (f) in the aqueous two-phase coacervate composition of the present invention. A non-limiting suitable example is Cremophor EL, a polyethoxylated castor oil commercially available from BASF Corporation prepared by reacting 35 moles of ethylene oxide with each mole of castor oil and which may include minor amounts of the polyethylene glycol esters of ricinoleic acid and polyethylene glycol ethers of glycerol.

The water-soluble biocompatible carrier present as a component (c) or (g) in the aqueous gel-free two-phase coacervate compositions of the present invention may be selected from a broad class of chemical compounds well known to the skilled person, e.g. from the group consisting of maltodextrins and polyols. Suitable polyols include monomeric polyols having at least 3 free hydroxyl groups and a molecular weight ranging from 100 to 350, and oligomeric polyols having at least 6 free hydroxyl groups and a molecular weight ranging from 350 to 3,000, in particular a molecular weight ranging from 350 to 1,800. Suitable examples thereof include acyclic polyols having from 3 to 12 carbon atoms and from 3 to 9 hydroxyl groups such as, but not limited to, erythritol (2R,3S-butane-1,2,3,4-tetraol), xylitol (2R,3R,4S-pentane-1,2,3,4,5-pentanol), sorbitol (2R,3S,4S,5S-hexane-1,2,3,4,5,6-hexol), mannitol (2R,3R,4R,5R-hexane-1,2,3,4,5,6-hexol), and mixtures thereof in any suitable proportions. Other suitable examples include cyclic or partly cyclic polyols having from 6 to 12 carbon atoms and from 5 to 9 hydroxyl groups such as, but not limited to maltitol (4-O-α-D-gluco-pyranosyl-D-glucitol), isomaltitol, lactitol (4-O-α-D-galacto-pyranosyl-D-glucitol), the cyclic isomers of fructose (D-fructofuranose and D-fructopyranose), and mixtures thereof in any suitable proportions. Other suitable examples include polyhydroxyketones such as the acyclic isomers of fructose (D-fructose and L-fructose). Other suitable examples include fructose oligomers, also known as fructooligosaccharides or oligofructose or oligofructan, which can be produced by enzymatic or chemical degradation of inulin and typically contain up to 7 fructose repeating units such as, but not limited to, kestose and inulobiose (dimers), nystose, bifurcose and inulotriose (trimmers), fructosylnystose and inulotetraose (tetramers) and the like, and mixtures thereof in any suitable proportions. Other suitable examples include fructose polymers, also known as polyfructose or inulin, being polymers of D-fructose residues linked by β(2-1) bonds with a terminal α(1-2) linked D-glucose and with a degree of polymerization from 8 to 60, such as but not limited to products marketed by Orafti N.V. (Tiene, Belgium) and Cosun (Netherlands). Another illustrative but less preferred polyol is glycerol (propan-1,2,3-triol).

Any bio-compatible or pharmaceutically acceptable grade of maltodextrins may be used as component (c) or (g) in the aqueous gel-free two-phase coacervate compositions of the present invention. Maltodextrin is a polysaccharide that is produced from starch and usually found as a hygroscopic powder. Maltodextrins are typically constituted of mixtures of chains that vary from 3 to 19 glucose units long and have a molecular weight below 1,800. Maltodextrins can be derived from any starch, e.g. from rice, corn, potato or wheat, and may contain traces of amino acids, including glutamic acid, or monosodium glutamate, as a manufacturing by-product.

As is well known to the skilled person, the different commercially available types of maltodextrins are mainly defined with respect to the two following parameters:
- the amylose and amylopectin contents of maltodextrin; in practice the amylose content commonly ranges from about 0.1 to about 70% by weight of maltodextrin and the amylopectin content commonly ranges from about 30 to about 99.9% by weight of maltodextrin; and
- the dextrose equivalent (herein referred as DE) of maltodextrin, which commonly ranges from about 1 to about 30, e.g. from 5 to 20, or from 9 to 27, or from 13 to 20.

The water-soluble biocompatible carrier present as a component (c) or (g) in the aqueous gel-free two-phase coacervate compositions of the present invention is readily distinguishable from the component (b) or (f) as it does not exhibit tensio-active properties.

According to another aspect, the present invention relates to the coacervate phase of an aqueous gel-free two-phase coacervate composition as defined in any of the specific embodiments described hereinabove. This coacervate phase can readily be separated (using techniques known in the art for liquid separation), if desired for further handling such as drying into a powder form, from the equilibrium water phase of the coacervate composition. The coacervate phase is of major industrial interest since, as noted above, it includes the major part of the poorly water-soluble biologically-active agent (a) or (e). According to a particular embodiment of this invention, from 70% to 100% of the bio-active agent (a) or (e) is dissolved in the coacervate phase. According to another particular embodiment of this invention, from 75% to 95% of the bio-active agent (a) or (e) is dissolved in the coacervate phase.

According to another aspect, the present invention relates to the equilibrium water phase of the coacervate composition (as defined in any of the specific embodiments described hereinabove). The equilibrium water phase can readily be separated (using techniques known in the art for liquid separation) from the coacervate phase of said gel-free two-phase coacervate composition. Although, as noted above, the equilibrium water phase includes a minor part, usually a very minor part (for instance from 5 to 25%), of the poorly water-soluble biologically-active agent (a) or (e), it may be useful as such under special circumstances.

In many situations, especially due to the easiness and cost-effectiveness of handling, long-term storage and transportation, it may be desirable to provide the formulation of the poorly water-soluble biologically-active agent (a) or (e) in solid form rather than in liquid form. Therefore, according to another aspect, the present invention relates to a powder being a dried form of the coacervate phase separated from the aqueous gel-free two-phase coacervate composition (as defined in any of the specific embodiments described hereinabove). According to yet another aspect, the present invention relates to a powder being a dried form of the aqueous gel-free two-phase coacervate composition itself, as defined in any of the specific embodiments described hereinabove. It has been unexpectedly found that these solid forms can be produced as free-flowing powders which will not cause handling problems after a substantial storage period. These solid forms can be produced using standard drying technology in the art such as, but not limited to, spray-drying or freeze-drying.

Spray drying suitable for this invention includes drying the aqueous two-phase coacervate composition as defined in any of the specific embodiments described hereinabove, or the coacervate phase thereof, through a hot gas. Typically, this hot gas may be air or, if oxygen-free drying is required, nitrogen gas. This spray-drying is a one step rapid process which eliminates the need for additional processing. The coacervate composition is pumped through an atomizer device that produces fine droplets into the main drying chamber. Atomizers vary with rotary, single fluid, two-fluid, and Ultrasonic Nozzle designs. The hot drying gas may be passed as a co-current or counter-current flow to the atomizer direction. A co-current flow usually enables the particles to have a lower residence time within the system and the particle separator (typically a cyclone device) to operate more efficiently. A counter-current flow enables a greater residence time of the particles in the chamber and may be paired with a fluidized bed system.

Any type of spray-dryer taking the aqueous liquid stream and quickly separating the solute or suspension as a solid (which may be collected in a drum or cyclone) and water into a vapor may be used. The liquid input stream is sprayed through a nozzle to make the droplets as small (e.g. from about 20 µm to about 200 µm) as possible, thus maximizing heat transfer and the rate of water vaporization. Suitable nozzle types include, but are not limited to, the following:
- the Rotary atomizer: located in the centre of the chamber roof. An air turbine drive operating from a sixbar compressed air source, supplies power to the atomizer wheel. A vaned atomizer wheel is used for non-abrasive feeds and an atomizer wheel with carbide bushings for atomizing abrasive feeds. Particles in the particle size range of 5-25 µm are created;
- the co-current two-fluid nozzle: located also in the centre of the chamber roof. The atomization is created by compressed air at a pressure of 0.5-2.0 bar. The feed and atomizing gas are passed separately to the nozzle head where the atomization takes place. Two-fluid nozzles have the advantage of handling more viscous feeds. The co-current mode is selected for drying heat sensitive products to fine particles in the particle size range of 5-25µm;
- the fountain two-fluid nozzle: located in the cone of the drying chamber, spraying upwards. The fountain mode is selected to meet a more coarse and free-flowing powder specification. Often used for fine ceramics and other non-heat-sensitive products. The counter-current flow offers a dryer performance with excellent heat utilisation and provides the longer powder residence time in the drying chamber required to complete the drying to larger particles in the particle size range of 15 - 80 µm.

Freeze-drying suitable for this invention includes freezing the aqueous two-phase coacervate composition as defined in any of the specific embodiments described hereinabove, or the coacervate phase thereof, and then reducing the surrounding pressure and adding enough heat to allow the frozen water in the coacervate to sublime directly from the solid phase to gas.

There are usually three steps in the complete freeze-drying process of this invention: freezing, primary drying, and secondary drying.

The freezing step consists of cooling the coacervate composition below its eutectic point, i.e. the lowest temperature at which the solid and liquid phases of the coacervate system can co-exist, to ensure that sublimation rather than melting will occur in the following steps. The coacervate system may be frozen slowly, or can be cycled up and down in temperature (annealing). Usually, the freezing temperature may be between about -50°C and -80 °C.

During the primary drying step, pressure is decreased (e.g. to a few millibars), and enough heat is supplied to the frozen coacervate for its water to sublimate. In this initial drying phase, about 95% of the water in the frozen coacervate is sublimated. This step may be slow (up to several days on industrial scale) in order to avoid alteration of the material's structure. In this step, pressure is controlled through the application of partial vacuum. The vacuum speeds sublimation, making it useful as a deliberate drying process. A cold condenser chamber and/or condenser plates (typically below -50 °C) provide a surface for the water vapor to re-solidify on, and prevents water vapor from reaching the vacuum pump, which could degrade the pump's performance.

The secondary drying step aims to remove any unfrozen water molecules, since ice was removed in the primary drying step. In this step, the temperature is raised higher than in the primary drying step, and can even be above 0°C, to break any physico-chemical interactions between the water molecules and the frozen material. Pressure may also be lowered (typically in the range of microbars) in this step to encourage desorption. After the freeze-drying process is complete, the vacuum is usually broken with an inert gas, such as nitrogen, before the resulting powder (with final residual water content typically around 1% to 4%) is sealed.

With each powder production method, the bio-active agent proportion in the composition is raised due to the removal of water. Starting from a coacervate composition within the scope of the first embodiment of this invention (low drug loading), the weight proportion of the poorly water-soluble biologically-active agent (a) in the resulting powder may range from about 3% to about 30%. Starting from a coacervate composition within the scope of the second embodiment of this invention, the weight proportion of the poorly water-soluble biologically-active agent (e) in the resulting powder may range from about 12.5% to about 50%.

The solid dosage form of the present invention may, especially when the poorly water-soluble biologically-active agent (a) or (e) is a therapeutic drug for human or veterinary use, also be in the form of granules which may be obtained by a method comprising a step of separating the coacervate phase from the equilibrium water phase of the aqueous gel-free two-phase coacervate composition, and using said coacervate phase as a granulating liquid for wet granulating a biologically-inactive carrier prior to a drying step which may be carried out at moderate temperature (including room temperature) under low relative humidity conditions for a relatively long period of time (ranging for instance from about 6 to 30 hours), or at a higher temperature (e.g. 40° to 60°C) for a shorter period of time (ranging for instance from about 1 to 6 hours). Exemplary but non-limiting biologically-inactive, pharmaceutically or veterinary acceptable carriers suitable for this purpose include microcrystalline cellulose; lactose; maltodextrins with a molecular weight below 1800 and/or a dextrose equivalent from 1 to 30; starch and pre-gelatinized starch; dicalcium and monocalcium phosphate; saccharose; fructose; sorbitol; and mannitol.

The respective weight proportions of the coacervate phase and the biologically-inactive carrier(s) in the above granule dosage form embodiment of the present invention may vary within relatively broad limits depending upon parameters such as, but not limited to, the type of therapeutic drug, and the type of biologically-inactive carrier. In any circumstance, such weight proportions may be determined by the skilled person without inventive effort and merely performing routine experimentation. For instance, the proportions may be from about 40% to about 80% by weight of the biologically-inactive carrier(s) and from about 20% to about 60% by weight of the coacervate phase.

Both the powder solid dosage form and the granule solid dosage form described hereinabove as being obtained from the first and second embodiments of the aqueous gel-free two-phase coacervate compositions of the present invention are able to provide optimal release of the poorly water-soluble biologically-active agent (a) or (e) from an aqueous medium within a short period of time, e.g. within no more than about 4 hours *in vivo,* or within no more than 10 minutes *in vitro.*

In many situations including, but not limited to, situations where the poorly water-soluble biologically-active agent (a) or (e) is a veterinary drug intended for mass medication, it may be desirable to provide the drug formulation in a drinking liquid form.

Unless exceptional circumstances where the aqueous gel-free two-phase coacervate composition can be produced at the place of bio-active agent consumption and soon or immediately before consumption, providing the biologically-active agent formulation in a liquid form may usually be suitably effected by dissolving or dispersing in water (or an essentially aqueous medium) the powder form that was previously obtained (e.g. by drying, spray-drying or freeze-drying), from the aqueous gel-free two-phase coacervate composition as described hereinabove, or the coacervate phase separated therefrom. Therefore, according to yet another aspect, the present invention relates to a water-soluble or water-dispersible formulation comprising a water-soluble amount, respectively a water-dispersible amount, of a powder or granule being a dried form of the aqueous two-phase coacervate composition as defined in any of the specific embodiments described hereinabove, or the coacervate phase thereof.

One critical advantage of the present invention is the ability to turn a poorly water-soluble biologically-active agent (a) or (e) into a solid form, e.g. a free-flowing powder form or a granule form, which is at least easily water-dispersible and even most often water-soluble. This water-solubility may depend upon parameters such as the exact nature and type of the poorly water-soluble biologically-active agent (a) or (e), the weight proportions of said biologically-active agent and the other components of the coacervate composition, the average particle size and/or the size polydispersity of the powder form, the type and operating conditions (e.g. temperature, pressure, and/or residence time) of the drying process used, and the temperature at which dissolution is performed. The determination of this water-solubility is a trivial exercise for the skilled person using routine experimentation.

Water-soluble or water-dispersible formulations as defined hereinabove are highly suitable for administration or application to a wide range of living species where the biologically-active agent (a) or (e) is potentially useful for treating or preventing a disorder or disease condition or for maintaining or improving health status. When the biologically-active agent (a) or (e) is a therapeutic drug or a cosmetic agent, the coacervate powder or granule form, or the water-soluble or water-dispersible formulation obtained therefrom, may be administered to human beings. When the biologically-active agent (a) or (e) is a veterinary drug, the coacervate powder or granule form, or the water-soluble or water-dispersible formulation obtained therefrom, may be administered to animals such as a mammal, a fish, a reptile or a bird. When the biologically-active agent (a) or (e) is a pesticide, a fertiliser or a herbicide, the coacervate powder or granule form, or the water-soluble or water-dispersible formulation obtained therefrom, may be applied onto plants such as crops, e.g. using spraying techniques well known in agriculture and horticulture.

According to yet another aspect, the present invention relates to a first method for producing an aqueous gel-free coacervate composition as defined herein (first or second embodiment), said method comprising the steps of:
(i) dissolving or dispersing the poorly water-soluble biologically-active agent (a) or (e) into the one or more non-ionic tensio-active agents (b) or (f) at a temperature comprised between 30°C below the melting point and 50°C above the melting point of said poorly water-soluble biologically-active agent (a) or (e);
(ii) adding the liquid solution obtained in step (i) to water under mixing, and
(iii) adding the one or more water-soluble carriers (c) or (g) to the aqueous mixture obtained in step (ii) under mixing.

According to a specific embodiment of this first method, step (i) is performed at a temperature comprised between 5°C above the melting point and 30°C above the melting point of the poorly water-soluble biologically-active agent (a) or (e). The non-ionic tensio-active agents (b) or (f) is preferably selected to be a liquid at the temperature used in step (i), i.e. to exhibit a melting point below said temperature.

According to yet another aspect, the present invention relates to an alternative method for producing an aqueous gel-free coacervate composition as defined herein (first or second embodiment), said method comprising the steps of:
(i) dissolving one or more water-soluble carriers (c) or (g) into water;
(ii) dissolving or dispersing the poorly water-soluble biologically-active agent (a) or (e) into the one or more non-ionic tensio-active agents (b) or (f) at a temperature comprised between 30°C below the melting point and 50°C above the melting point of said poorly water-soluble biologically-active agent (a) or (e), and
(iii) adding the liquid solution or dispersion obtained in step (ii) to the solution prepared in step (i) under mixing.

According to a specific embodiment of this second method, step (ii) is performed at a temperature comprised between 5°C above the melting point and 30°C above the melting point of the poorly water-soluble biologically-active agent (a) or (e). The non-ionic tensio-active agents (b) or (f) is preferably selected to be a liquid at the temperature used in step (ii), i.e. to exhibit a melting point below said temperature. According to another specific embodiment of this second method, step (i) is performed at a temperature at which the water-soluble carrier (c) or (g) is easily soluble in water, with or without stirring, for instance a temperature from 15°C to 80°C.

As is readily understandable by the skilled person, the two above methods distinguish from each other by the order in which the required components of the aqueous coacervate composition are mixed. Depending upon parameters such as, but not limited to, the exact nature and the respective amounts of the poorly water-soluble biologically-active agent (a) or (e), the non-ionic tensio-active agents (b) or (f), and the water-soluble carriers (c) or (g), one method may prove to be more time-efficient or more convenient in maximising the amount of biologically-active agent (a) or (e) retained in the coacervate phase of the two-phase composition.

Since both methods are suitable for producing aqueous coacervate compositions as defined in any of the first and second embodiments described hereinabove, the weight amounts of the poorly water-soluble biologically-active agent (a) or (e), the non-ionic tensio-active agents (b) or (f), the water-soluble carriers (c) or (g), and water (d) or (h) introduced in the relevant steps (i), (ii) and (iii) are such as defined in said respective embodiments.

Both methods may further comprise a step of cooling down the mixture until the coacervate phase separates from the equilibrium water phase. Such cooling step may be spontaneous or may be accelerated through the use of suitable cooling means.

Although in some situations, a high shear rate of mixing (e.g. with rotational agitation at a rotation speed of about 5,000 to 10,000 rpm) has proven to be efficient in performing steps (ii) and/or (iii) of the first method, or in step (iii) of the alternative method, this type of mixing is not a requirement of the methods of this invention. Depending upon parameters such as, but not limited to, the respective amounts of the poorly water-soluble biologically-active agent (a) or (e), the non-ionic tensio-active agents (b) or (f), the water-soluble carriers (c) or (g), and water (d) or (h), both methods may also be performed at lower shear rates. Suitable high shear homogenizers include, but are not limited to, the Silverson Mixer Model L4R, a multi-purpose high shear mixer suitable for the widest range of applications - mixing, emulsifying, homogenising, dissolving - with high efficiency and flexibility, with a capacity up to 12 litres and the ability to mix in-line with various flow rates, and offering a full load speed of 6000 to 8000 rpm.

Since in each production method the three designated steps have proven to be sufficient for achieving aqueous coacervate compositions with suitable behaviour, each production method may consist of, rather than comprise, these three steps.

The following examples are provided solely for the purpose of illustrating various embodiments of the present invention, and without any intention of limiting the scope thereof.

### Example 1 - preparation of an aqueous gel-free coacervate system based on Cremophor EL

150 g Cremophor^{®} EL (a polyoxyl 35 castor oil non-ionic tensio-active agent commercially available from BASF Corporation) was heated to 150°C and 60 g febantel (melting point: 127-132°C) was dissolved in the heated liquid. When the drug was visually dissolved, the solution was kept at this temperature for 2 minutes. Thereafter, the warm solution was added to 1000 ml demineralised water under high shear mixing (using a Silverson L4R device operated at 6000-8000 RPM) and after addition, mixing was continued during 5 minutes. Thereafter, 300 g maltodextrin (dextrose equivalent 18, commercially available as C*PharmDry 01983 from Cargill, France) was slowly added to the liquid under high-shear conditions (using a Silverson L4R device operated at 6000-8000 RPM) and mixed during 5 minutes. After a cooling down period of 8 hours, an upper coacervate phase and a lower equilibrium water phase were obtained.

### Example 2 ― production and water-dissolution of a febantel powder

After dispersing the coacervate phase into the equilibrium water phase of the coacervate composition prepared in example 1 by shaking and/or stirring, the formulation was spray-dried to a white free-flowing powder by means of a laboratory scale spray-drying equipment Mobile Minor commercially available from Niro, Copenhagen, Denmark. The feed rate of the dispersed liquid was set at 30 ml/minute, the inlet temperature at 130°C and the resulting outlet temperature was 58 °C.

A dissolution test in water was performed for the spray-dried free-flowing powder formulation at room temperature (20 - 25°C). The test showed that the drug powder formulation was completely dissolved within 5 minutes.

### Example 3 - preparation of an aqueous gel-free coacervate system based on Tween 80

200 g Tween^{®} 80 was heated to 150°C and 60 g febantel was dissolved in the heated liquid. When the drug was visually dissolved, the solution was kept at this temperature for 2 minutes. Thereafter, the warm solution was added to 1000 ml demineralised water under high shear mixing conditions (using a Silverson L4R device operated at 6000-8000 RPM) and after addition, mixing was continued during 5 minutes. Thereafter, 400 g maltodextrin (dextrose equivalent 18, commercially available as C*PharmDry 01983 from Cargill, France) was slowly added to the liquid under high-shear forces (using a Silverson L4R device operated at 6000 - 8000 RPM) and mixed during 5 minutes and stirred afterwards. An upper coacervate phase and a lower equilibrium water phase were obtained.

### Example 4 - production and water-dissolution of a febantel powder

After dispersing the coacervate phase into the equilibrium water phase of the coacervate composition prepared in example 3 by means of shaking and/or stirring, the formulation was spray-dried to a white powder by means of a laboratory scale spray-drying equipment Mobile Minor commercially available from NIRO, Copenhagen, Denmark. The feed rate of the dispersed liquid was set at 28 ml/minute, the inlet temperature at 130°C and the resulting outlet temperature was 60 °C.

A dissolution test in water was performed at room temperature (20 - 25°C). The test showed that the drug powder formulation was completely dissolved within 5 minutes.

### Example 5 (comparative) - preparation of a physical mixture based on Cremophor EL

6 g febantel was mixed with 30 g maltodextrin (dextrose equivalent 18, commercially available as_C*PharmDry 01983 from Cargill, France) by using the geometric dilution method, i.e. an equal amount (6 g) of maltodextrin was mixed with the total amount of febantel. An equal amount (12 g) of maltodextrin was mixed with the first mixture and so on. Thereafter, 15 g Cremophor^{®} EL (a polyoxyl 35 castor oil non-ionic tensio-active agent commercially available from BASF Corporation) was used as a granulation liquid for the mixture of febantel and maltodextrin.

### Example 6 - comparison of aqueous dispersions of a spray-dried coacervate powder and a physical mixture based on Cremophor EL

When the spray-dried powder of example 2 was dispersed in water under stirring, a clear liquid was observed. The particles were floating in water and a very small amount of particles was precipitated on the bottom of the beaker. After 5 minutes the liquid was completely clear and all floating and precipitated particles were disappeared.

When the physical mixture of example 5 was dispersed in water under stirring, a clear liquid was observed. The particles were floating in the water and a significant amount of particles was precipitated on the bottom of the beaker. The floating particles disappeared after 5 minutes but, in contrast to the previous observation, a fraction of precipitated particles was still observed even after 60 minutes.

The same dissolution test in water was performed at room temperature (20-25°C) as in example 2. The test showed that only 3% of drug from the physical mixture was dissolved after 60 minutes, while (as noted in example 2) the spray dried formulation was completely dissolved within 5 minutes.

### Example 7 - storage stability of a coacervate formulation

This example illustrates the stability of the coacervate powder formulation of example 2 at 7°C. A dissolution test was performed in water at room temperature (T = 20,9 °C). Afterwards, the samples were stored at 7°C and samples were taken immediately after dissolution, and 1, 7 or 14 days after storage of the dissolved formulation at 7 °C. The percentage of drug recovered from the samples remains constant even after 14 days of storage at 7°C.

### Example 8 - X-ray characterization of a spray-dried coacervate formulation of febantel

The physical characteristics of the spray-dried powder obtained from the coacervate composition of example 2 was investigated by X-ray diffraction. Due to the absence of peaks in the X-ray diffraction pattern, it can be said that no febantel crystals are present in the spray-dried powder.

### Example 9 - determination of febantel proportion in the coacervate phase

This example illustrates the amount of drug which can be achieved in the coacervate phase, as measured indirectly onto the coacervate system of example 1.

The amount of febantel in the equilibrium water phase was first determined. Therefore, a sample of 10 ml was taken from the equilibrium water phase and a volume of 200 µl was diluted in 20 ml methanol and sonicated for 15 minutes. Thereafter, a test tube of 10 ml was filled with this mixture and centrifuged for 5 minutes at 4000 RPM. Then, a sample of 500 µl was taken and further diluted to 10 ml with methanol. The absorbance of this sample was measured by UV spectroscopy operating a double beam spectro-photometer (Shimadzu UV-1650 PC, Kyoto, Japan) at a wavelength of 280 nm. Based on the absorbance and the total volume of the liquid phase, the concentration of febantel was calculated, indicating that 10% of the drug was retained in the equilibrium water phase. As a consequence this means that 90 % of febantel was retained in the coacervate phase.

### Example 10 - alternative production method for the coacervate system

This example illustrates an alternative method for producing a coacervate composition of this invention.

30 g maltodextrin (dextrose equivalent 18, commercially available as C*PharmDry 01983 from Cargill, France) was dissolved in 100 ml of water. Thereafter 15 g Cremophor^{®} EL (commercially available from BASF Corporation) was heated to 150°C and 6 g febantel was dissolved in the heated Cremophor^{®} EL. After the drug was visually dissolved, the solution was kept at temperature (150°C) for 2 minutes. These solution was added to water containing the dissolved maltodextrins under high shear mixing (using a Silverson L4R device operated at 6000-8000 RPM) and after addition, these compounds were mixed during 5 minutes.

In this way of production, a coacervate phase and an equilibrium water phase were obtained as well.

### Example 11 - alternative aqueous coacervate composition

This example illustrates the influence of using a mixture of water-soluble carriers. 15 g Cremophor^{®} EL (commercially available from BASF Corporation) was heated to 150 °C and 6 g febantel was dissolved in the heated liquid. When the drug was visually dissolved, the solution was kept at temperature for 2 minutes. Thereafter, the mixture febantel - Cremophor^{®} EL was added to 100 ml demineralised water under high shear mixing conditions (using a Silverson L4R device operated at 6000-8000 RPM) and after addition, these compounds were mixed during 5 minutes. Thereafter, a mixture of 20 g maltodextrin (dextrose equivalent 18, commercially available as C*PharmDry 01983 from Cargill, France) and 10 g mannitol was slowly added to the liquid under high-shear forces and mixed (using a Silverson L4R device operated at 6000-8000 RPM) during 5 minutes. A coacervate phase and an equilibrium water phase were obtained as well.

### Example 12 - preparation of a high drug loading aqueous gel-free coacervate system based on Cremophor EL

150 g Cremophor^{®} EL (a polyoxyl 35 castor oil non-ionic tensio-active agent commercially available from BASF Corporation, New Jersey) was heated to 150°C and 60 g febantel (melting point: 127-132°C) was dissolved in the heated liquid. When the drug was visually dissolved, the solution was kept at this temperature for 2 minutes. Thereafter, the warm solution was added to 80 ml demineralised water under high shear mixing (using a Silverson L4R device operated at 6000-8000 RPM) and after addition, mixing was continued during 5 minutes. Thereafter, 10 g maltodextrin (dextrose equivalent 18, commercially available as C*PharmDry 01983 from Cargill, France) was slowly added to the liquid under high-shear conditions (using a Silverson L4R device operated at 6000-8000 RPM) and mixed during 5 minutes. After a cooling down period of 8 hours, an upper coacervate phase and a lower equilibrium water phase were obtained.

### Example 13 - alternative production method for the coacervate system

60 g febantel, a poorly water-soluble drug (solubility 0.019 mg/mL, melting point 129.5°C), was dissolved in 150 g of a molten polyoxyl 35 castor oil non-ionic surfactant (Cremophor^{®} EL, commercially available from BASF Corporation, New Jersey) heated at 130°C. This molten phase was kept at 130°C for 2 minutes before being added to 1 liter of an aqueous solution (30% m/v) of maltodextrin (dextrose equivalent 18, commercially available as C*PharmDry 01983 from Cargill, France) under homogenization (8000 rpm for 10 minutes) using a high sheer mixer (Silversson L4R, Waterside, UK). A milky liquid system was obtained, which was left to cool down for a period of 8 hours during which a coacervate biphasic system including an upper phase and a lower equilibrium water phase was formed.

### Example 14 - determination of febantel proportions in the coacervate system

The lower phase of the biphasic system of example 13 was mainly composed of maltodextrin and febantel (1% of the drug amount), as quantified by UV spectroscopy operating a double beam spectro-photometer (Shimadzu UV-1650 PC, Kyoto, Japan) at a wavelength of 280 nm. Maltodextrin was not detected in the coacervate upper layer which was composed of febantel (99% of the drug amount) and Cremophor®EL.

### Example 15 - production of a spray dried febantel powder formulation

The biphasic coacervate system of example 13 was re-dispersed using a paddle homogenizer (IKAWERK, Germany) and spray dried using a laboratory scale spray-drying equipment Mobile Minor commercially available from NIRO, Copenhagen, Denmark. This equipment was operated in a co-current air flow with a inlet temperature of 130°C, an outlet temperature of 60°C and a liquid feed rate of 20-30 ml/minute. From this process a free flowing powder of febantel was obtained with a 10% w/w drug loading.

### Example 16 - production of a granulated febantel formulation

The febantel-rich upper phase of the biphasic coacervate system of example 13 was separated from the lower aqueous phase and used as granulation fluid for the production of microcrystalline cellulose-based granules (160 mL coacervate phase mixed with 300 g of microcrystalline cellulose commercially available under the tradename AVICEL PH 101). The granules were formed by passing the mixture through a 1400 mesh sieve, then dried for 12 hours in a controlled humidity room (< 20% relative humidity). The resulting granules contained 10% by weight febantel.

### Example 17 - characterization of febantel powder formulations by means of a dissolution test

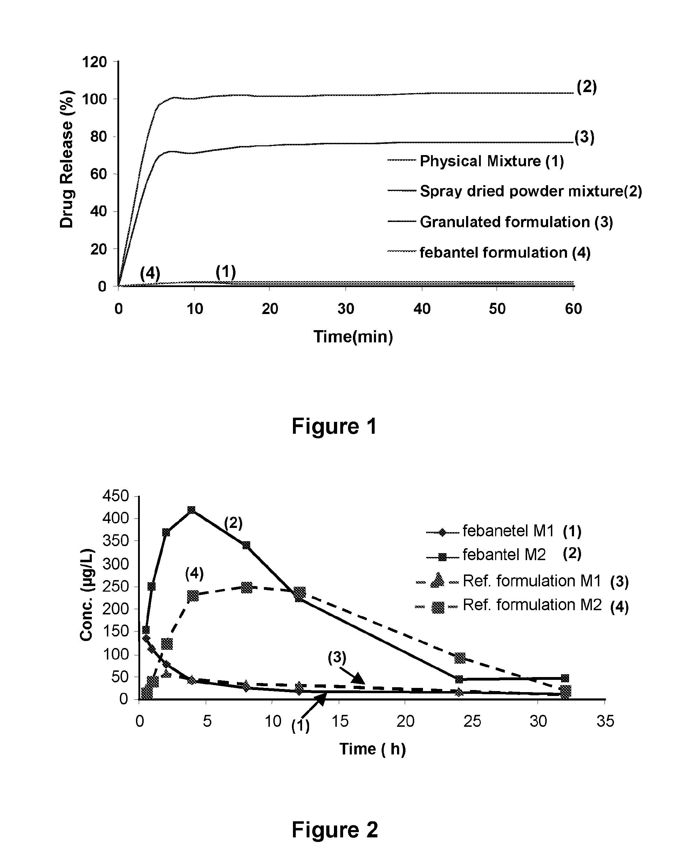

The spray-dried febantel powder formulation of example 15, the granulated febantel formulation of example 16, the comparative physical mixture of example 5, and a commercially available febantel reference formulation were submitted to a dissolution test according to U.S. Pharmacopeia standard 27 on a Vankel VK7010 dissolution tester combined with a VK8000 automatic sampling station. Dissolution testing was performed in water at room temperature. Samples of 5 mL were withdrawn from the dissolution vessels at 5, 10, 15, 20, 30, 45 and 60 minutes respectively. Results are shown in figure 1. In contrast with both the comparative physical mixture of example 5 and the commercially available febantel reference formulation, febantel was rapidly released from the powder and granule formulations of examples 15 and 16. Without wihing to be bound by theory, the lower febantel release from the granule formulation may be due to the adsorption of febantel onto microcrystalline cellulose.

### Example 18 ― storage stability of coacervate formulations under different temperature and humidity conditions

The storage stability of the powder and granule formulations of examples 15 and 16 was investigated. Storage at 25°C and 60% relative humidity (RH), or at 40°C and 75% RH, did not significantly change the febantel release profile in the dissolution test of example 17. From the spray dried powder formulation of example 15, 100% release was achieved within 10 minutes. No solid state changes of the drug were observed during storage under such conditions.

### Example 19 ― in vivo evaluation of febantel formulations in pias

The spray-dried febantel powder formulation of example 15 and a commercially available febantel reference formulation were submitted to a crossover study in 6 pigs (20-30 kg, ± 9 weeks old). Febantel-containing formulations were orally administered, and blood samples were taken at 0.5, 1, 2, 4, 8, 12, 24, 32 hours after administration of the respective formulation. Febantel is known to be metabolized into two active metabolites (fenbendazole and oxfendazole respectively, hereinafter denoted M1 and M2) the plasma concentrations of which were determined. Results are shown in figure 2. The maximum plasma concentrations of both metabolites were much higher after administration of the spray-dried powder formulation of example 15 compared to the commercially available product.

### Example 20 - alternative coacervate composition

525 g Cremophor EL was heated to 100°C and 60 g of ketoprofen (melting point 94°C) was dissolved in the heated liquid. When the drug was visually dissolved, the solution was kept at this temperature for 2 minutes.

Thereafter the warm solution was added to 1,000 g demineralised water under high shear mixing (Silverson L4R device operated at 6000 - 8000 rpm and the mixing continued for 5 minutes. Thereafter 300 g maltodextrin (C° Pharm Drug 01983 from Cargill, France) was slowly added to the liquid under high shear conditions (Silverson L4R device operated at 6000-8000 rpm and mixed during 5 minutes. After a cooling down period of 6 hours, a two-phase coacervate composition comprising an upper coacervate phase and a lower equilibrium water phase was obtained.

### Example 21 - production and water-dissolution of a ketoprofen powder

After dispersing the coacervate phase into the equilibrium water phase of the coacervate composition of example 20 by means of shaking and/or stirring, the formulation was spray dried using a laboratory scale spray-drying equipment Mobile Minor commercially available from Niro, Denmark. The feed rate of the dispersed liquid was set at 25 ml/minute, the inlet temperature at 115°C and the resulting outlet temperature was 55°C.

A dissolution test using the paddle dissolution testing device as described in Ph.Eur.of the resulting spray dried ketoprofen powder formulation in water was performed at 20°C. The test showed that the drug powder formulation was completely dissolved within 5 minutes.

### Example 22

510 g Tween 80 was heated to 110°C and 45 g fenofibrate (melting point 80°C) was dissolved in the heated liquid and the solution was kept at this temperature for 2 minutes.

Thereafter the warm solution was added to 1,000 g demineralised water under high shear mixing (Silverson L4R device operated at 6000-8000 rpm) and continuously mixed for 5 minutes. Thereafter 200 g mannitol was slowly added during 5 minutes. After a cooling down period of 6 hours, a two-phase coacervate composition comprising an upper coacervate phase and a lower equilibrium water phase was obtained.

### Example 23 - production and water-dissolution of a fenofibrate powder

After dispersing the coacervate phase into the equilibrium water phase of the coacervate composition of example 22 by means of shaking and/or stirring, the formulation was spray dried (using the same equipment as in example 21) . The feed rate was set at 15 ml/minute, the inlet temperature at 120°C, and the resulting outlet temperature was 60°C.

A dissolution test using the paddle dissolution testing device as described in Ph. Eur. of the resulting spray dried fenofibrate powder formulation in water was performed at 37°C. The test showed complete dissolution of the drug powder formulation within 20 minutes.

### Example 24 - alternative coacervate composition

1,5 g indomethacin was dissolved in 30 g Lutrol L44 (a polyglycol with a molecular weight of 2,200 from BASF Canada Inc.) heated at 150°C by stirring the resulting molten phase during 5 minutes.

Separately, 30 g maltodextrin (DE 18) was dissolved in water. Then, the molten phase was added to the aqueous maltodextrin phase under homogenization conditions (8000 rpm for 10 minutes) by using a high shear mixer.

After a cooling down period of 6 hours, a two-phases coacervate composition comprising an upper coacervate phase and a lower equilibrium water phase was obtained.

### Example 25 - production of a spray dried indomethacin powder formulation

The biphasic coacervate system of example 24 was re-dispersed using the same homogenization conditions as in example 24, and then spray dried using the same equipment as in example 15 at an inlet temperature of 140°C, an outlet temperature of 90°C and a liquid feed rate of 20 ml per minute.

From this process a free flowing powder of indomethacin was obtained with a drug loading of 2.5 weight %.

### Example 26 - production of a granulated indomethacin formulation

The indomethacin-rich upper phase of the biphasic coacervate system of example 24 was separated from the lower aqueous phase and used as granulation fluid for the production of lactose-based granules. A mixture of lactose monohydrate (75% by weight) and the coacervate upper phase (25% by weight) were granulated in a twin screw extruder as the granulating tool. The resulting granules were then dried at 60°C using a fluid bed dryer.

### Example 27 - production of an alternative granulated indomethacin formulation

The indomethacin-rich upper phase of the biphasic coacervate system of example 24 was separated from the lower aqueous phase and used as granulation fluid for the production of lactose-based granules. A mixture of microcrystalline cellulose (50% by weight) and the coacervate upper phase (50% by weight) were granulated in a twin screw extruder as the granulating tool. The resulting granules were then dried at 60°C using a fluid bed dryer.

## Claims

1. An aqueous gel-free two-phase coacervate composition comprising a coacervate phase and an equilibrium water phase, wherein said composition comprises, per 100 parts by weight, a mixture of:
(a) from 2 to 10 parts by weight of a poorly water-soluble biologically-active agent,
(b) from 10 to 30 parts by weight of a tensio-active system with a HLB from 8 to 18 and consisting of one or more non-ionic tensio-active agents,
(c) from 10 to 25 parts by weight of one or more water-soluble carriers for said poorly water-soluble biologically-active agent, said carrier being selected from the group consisting of monomeric polyols having at least 3 free hydroxyl groups and a molecular weight ranging from 100 to 350, and oligomeric polyols having at least 6 free hydroxyl groups and a molecular weight ranging from 350 to 3,000, and
(d) from 40 to 70 parts by weight of water,
wherein the weight ratio (c)/(b) of the water-soluble carriers to the non-ionic tensio-active agents in the said mixture is from 0.3/1 to 2.5/1.

2. An aqueous gel-free two-phase coacervate composition comprising a coacervate phase and an equilibrium water phase, wherein said composition comprises, per 100 parts by weight, a mixture of:
(e) more than 10 parts and up to 30 parts by weight of a poorly water-soluble biologically-active agent,
(f) more than 30 parts and up to 70 parts by weight of one or more non-ionic tensio-active agents,
(g) from 2 to less than 10 parts by weight of one or more water-soluble carriers for said poorly water-soluble biologically-active agent, said carrier being selected from the group consisting of monomeric polyols having at least 3 free hydroxyl groups and a molecular weight ranging from 100 to 350, and oligomeric polyols having at least 6 free hydroxyl groups and a molecular weight ranging from 350 to 3,000, and
(h) from 20 to less than 40 parts by weight of water,
wherein the weight ratio (g)/(f) of the water-soluble carriers to the non-ionic tensio-active agents in the said mixture is from 0.005/1 to less than 0.3/1.

3. An aqueous two-phase coacervate composition according to claim 1 or claim 2, wherein said poorly water-soluble biologically-active agent is a therapeutically active drug, a pesticide, a cosmetic agent, a fertiliser, a herbicide, a plant growth regulator, a crop treatment agent or an anti-microbial agent.

4. An aqueous two-phase coacervate composition according to any one of claims 1 to 3, wherein said carrier is selected from the group consisting of erythritol, xylitol, sorbitol, mannitol, maltitol, isomalt, lactitol, fructose, fructose oligomers and polyfructose.

5. An aqueous two-phase coacervate composition according to any one of claims 1 to 3, wherein from 70% to 100% of said poorly water-soluble biologically-active agent is dissolved in the coacervate phase.

6. An aqueous two-phase coacervate composition according to any one of claims 1 to 5, wherein said non-ionic tensio-active agent is selected from the group consisting of cholesterol esters, ethylene glycol fatty acid esters, ethoxylated esters, (poly)glycerol fatty acid esters, mono- and diglycerides of fatty acids, poloxamers, polyoxyethylene alkyl ethers, polyoxyethylene castor oil derivatives, polyoxyethylene sorbitan fatty acid esters, propyleneglycol esters, sorbitan fatty acid esters, and mixtures thereof.

7. The coacervate phase separated from an aqueous two-phase coacervate composition according to any one of claims 1 to 6.

8. The equilibrium water phase separated from an aqueous two-phase coacervate composition according to any one of claims 1 to 6.

9. A powder being a dried form of the coacervate phase of claim 7 or the coacervate composition of claim 1 or claim 2.

10. A water-soluble formulation comprising a water-soluble amount of a powder according to claim 9.

11. A method for producing an aqueous coacervate composition according to claim 1 or claim 2, comprising the steps of:
(i) dissolving or dispersing the poorly water-soluble biologically-active agent into the one or more non-ionic tensio-active agents at a temperature comprised between 30°C below the melting point and 50°C above the melting point of said poorly water-soluble biologically-active agent;
(ii) adding the liquid solution obtained in step (i) to water under mixing, and
(iii) adding the one or more water-soluble carriers to the mixture obtained in step (ii) under mixing.

12. A method for producing an aqueous coacervate composition according to claim 1 or claim 2, comprising the steps of :
(i) dissolving one or more water-soluble carriers into water;
(j) dissolving or dispersing the poorly water-soluble biologically-active agent into the one or more non-ionic tensio-active agents at a temperature comprised between 30°C below the melting point and 50°C above the melting point of said poorly water-soluble biologically-active agent, and
(k) adding the liquid solution or dispersion obtained in step (j) to the solution prepared in step (i) under mixing.

13. A method for producing an aqueous coacervate composition according to claim 11 or claim 12, further comprising the step of cooling down the mixture until the coacervate phase separates from the equilibrium water phase.

14. A method for producing a powder according to claim 9, comprising the steps of :
(i) producing an aqueous two-phase coacervate composition according to claim 1 or claim 2;
(ii) optionally separating the coacervate phase from the equilibrium water phase of said aqueous two-phase coacervate composition, and
(iii) drying the coacervate phase, optionally combined together with the equilibrium water phase, until at least 70% of water contained in said phase(s) is removed.

15. A method according to claim 14, wherein the drying step (iii) is effected by spray-drying or freeze-drying.
